# EUROPEAN PATENT APPLICATION

(11) **EP 3 721 804 A1**
(43) Date of publication of application: **14.10.2020**
(21) Application number: 19168643.5
(22) Date of filing: 11.04.2019
(51) Int. Cl.: A61B 5/145, A61M 16/00

(54) **IDENTIFICATION AND QUANTIFICATION OF A VENTILATORY DISTUBANCE CAUSING INCORRECT MEASUREMENT OF ARTERIAL ACID-BASE STATUS**

(71) Applicant: Obi ApS, 9560 Hadsund (DK)
(72) Inventor: Shastri, Lisha, 9000 Aalborg (DK); Thomsen, Lars Pilegaard, 9520 (DK); Graversen, Bruno, 9560 Hadsund (DK); Rees, Stephen Edward, 9260 Gistrup (DK)
(74) Representative: Plougmann Vingtoft a/s

(57) **Abstract**

The present invention relates to a computer-implemented method for identification of the degree to which a transient disturbance in ventilation causes changes in measurement of arterial acid-base status.

## Description

### FIELD OF THE INVENTION

The present invention relates to a computer-implemented method for identification of the degree to which a transient disturbance in ventilation causes changes in measurement of arterial acid-base status. The method applies calculated arterial values of acid-base status obtained from the analysis of venous blood, along with measured values of arterial acid-base status. The differences between calculated and measured values are used to quantify the addition or removal of carbon dioxide necessary to account for transient disturbances in arterial acid-base status due to changes in ventilation. The invention also relates to a corresponding data processing system, and a corresponding computer program product for execution on a computer.

### BACKGROUND OF THE INVENTION

Patients with acute respiratory illness, often require an arterial blood gas to measure both oxygenation and values of acid-base status of arterial blood, including the pH and partial pressure of carbon dioxide (PCO₂). Blood samples are usually obtained by puncturing an artery, which is painful, particularly so in contrast to puncture of a vein. A method has therefore been previously developed (1,2 cited below) to calculate arterial values from measurements taken in venous blood. The method uses non-invasive measurements of arterial oxygenation, taken from a pulse oximeter using a finger or ear clip, and then calculates the addition of oxygen required to convert the venous oxygen values to arterial. By then assuming that the CO₂ removed so as to convert venous blood gases to arterial, is a fixed ratio of the oxygen added, the method calculates the complete arterial oxygen and acid-base profile. This fixed ratio can be used, as the production of CO₂ and consumption of O₂ in aerobic metabolism are directly related. This ratio is known as the respiratory quotient (RQ).

The method has been shown to function accurately in patients of a number of studies (3-6). The concerned method for converting venous blood values to arterial blood values is disclosed in the international patent application WO 2004/010861 (to OBI Medical Aps, Denmark). While it is well known that there are artefacts regarding the influence of ventilation on arterial blood, e.g., hyperventilation, hypoventilation, etc., the degree and quantification of these effects is poorly understood. Hence, an improved method to explain how a ventilation change can modify arterial acid-base status would be advantageous.

### OBJECT OF THE INVENTION

It is an object of the present invention to provide an alternative to the prior art. In particular, it may be seen as an object of the present invention to provide a method that solves the above mentioned problems of the prior art with identification of the degree to which transient changes in ventilation have modified measurements of the acid-base status of a subject's, or patient's, arterial blood.

### SUMMARY OF THE INVENTION

Thus, the above-described object and several other objects are intended to be obtained in a first aspect of the invention by providing a computer-implemented method for determining degree to which the arterial blood sample has been modified by the presence of a transient increase, or decrease, in ventilation of a subject, the method comprising
a) measuring and/or estimating values of acid-base status in a blood sample, the blood sample being obtained from venous blood of the subject,
b) providing values of measured and/or estimated arterial oxygenation from said subject,
c) converting the venous blood values by applying a venous-to-arterial conversion model for deriving blood acid-base status and oxygenation status into first estimated arterial blood values,
d) providing second reference acid-base status and oxygenation values of arterial blood from the said subject,
e) implementing a ventilation disturbance model using a measure of the total carbon dioxide content in the arterial blood, the said model having as an input, at least, said first estimated arterial blood values, and said second reference values of arterial blood,
f) the ventilation disturbance model calculating a measure indicative of the difference in the total carbon dioxide content between the said first estimated arterial blood values and said second reference values of arterial blood, and
g) the ventilation disturbance model being arranged to output a measure indicative of the degree to which the arterial blood sample has been modified by the presence of a transient change in ventilation using said measure.

The present invention is particularly - but not exclusively - advantageous, in that measurement and analysis, i.e. comparison, of measured and calculated values of arterial acid-base status can be applied to obtain hitherto unavailable insight about the changes in measurement of arterial acid-base status due to transient modifications in ventilation. Insights that - to the best knowledge of the inventors - was previously not available in this field.

Thus, the method of the invention provides an indication as to the degree of transient ventilation responsible for modification of measurements of arterial acid-base status, which may allow a clinician to take appropriate action, such as deciding to perform a second arterial measurement some time later when the transient change in ventilation has passed, so as to confirm the correct levels of arterial acid-base status.

In the broadest sense, the invention may be advantageously applied to assist in evaluating whether measured arterial values represent the patient's acid-base status in steady state conditions, or whether they have been modified by the effects of transient ventilation. The presence of modifications can be described in two-result fashion, i.e., 'present' or 'not present', but the invention may of course also output a more nuanced level of this risk, both qualitatively and in a quantified manner. Thus, in a quantitative manner it could be a number, such as a the differences in CO₂ or pH levels in the calculated and measured arterial values, or the CO₂ necessary to be added or removed so as to convert calculated arterial to measured values. If provided in a qualitative manner it could be e.g., a three-level regime, e.g. 'present', 'to a small extent' and 'to a large extent', a four-level risk regime, and so forth. Thus, in embodiment, the output measure indicative of a degree in which the arterial blood sample has been modified by the presence of a transient in the ventilation may be a quantitative measure, the quantitative measure subsequently being compared with a pre-defined limit for whether a transient in the ventilation has been detected, or not.

The degree to which arterial blood gas has been modified by transient changes in ventilation may be output and indicated to a user, e.g., a clinician, in any kind of suitable graphical user interface (GUI), by sounds/alarms, or other human-machine interfaces, and/or stored for later use, e.g., for analysis and assessment by a clinician.

It may be mentioned that - in the context of the present invention - the said indications are intended for assisting or guiding e.g., the clinician in making decisions of a therapeutic and/or diagnostic character. Thus, the present invention is not designed to make an actual diagnosis, but merely to provide intelligent information, i.e., indications that may assist the clinician in executing the subsequent step and making the intellectual exercise of providing a diagnosis for the patient and evaluation of the quality of the arterial acid-base measurements. The diagnosis may then be followed by an action of therapeutic character, if needed.

In one embodiment, the ventilation disturbance model may further be performing a minimization process of the measure so as find an optimum value of the first measure indicative of a possible transit change in the ventilation of the subject or patient. The skilled person will understand that mathematically the process of finding an optimum value of the measure could be performed by alternative mathematical methods, such as a reformulation to a maximization process, etc. Beneficially, the minimization process of the measure may performed by an iteration process using said measure, especially considering the normally complex mathematical formulas expressing the ventilation disturbance model.

In one embodiment, the iteration of the ventilation disturbance model may be mathematically modifying the total carbon dioxide content so as to approximate the first estimated arterial blood values to the second reference values of arterial blood. In an alternative embodiment, it may be opposite, i.e. the iteration of the ventilation disturbance model may be mathematically modifying the total carbon dioxide content so as to approximate the second reference values of arterial blood to the first estimated arterial blood values. It is contemplated, that if time and/or processing resources allows, it is possible to perform both iterations to perform a consistency or stability check of the iterations.

In another embodiment, two parameters indicative of the difference between pH and PCO₂, respectively, derived from said first estimated arterial blood values, and said second reference values of arterial blood, may be used for the minimization process. These two parameters may also be combined into a common error function for the minimization. As it is explained in more details below, this allows for an efficient way of finding the measure indicative of a transient change in ventilation having an influence on the arterial blood values of the subject.

In a second aspect, the invention relates to a data processing system for determining a degree in which the arterial blood sample has been modified by the presence of a transient increase, or decrease, in ventilation, said data processing system comprising:
a) means for measuring and/or estimating values of blood acid-base status in a blood sample, the blood sample being obtained from venous blood of the subject,
b) means for receiving, or providing, values of measured and/or estimated arterial oxygenation from said subject,
c) means for converting the venous blood values by applying a venous-to-arterial conversion model for deriving blood acid-base status and oxygenation status into first estimated arterial blood values,
d) means for receiving, or providing, second reference acid-base status and oxygenation values of arterial blood from the said subject,
e) means for implementing a ventilation disturbance model using a measure of the total carbon dioxide content in the arterial blood, the said model having as input, at least, said first estimated arterial blood values, and said second reference values of arterial blood,
f) wherein the ventilation disturbance model is calculating a measure indicative of the difference in the total carbon dioxide content between the said first estimated arterial blood values and said second reference values of arterial blood, and
g) the ventilation disturbance model is arranged to output a measure indicative of a degree to which the arterial blood sample has been modified by the presence of a transient change in the ventilation using said measure.

In a third aspect, the invention relates to a computer program product being adapted to enable a system comprising of at least one computer having means of data storage in connection therewith to control a data processing system according to the second aspect of the invention.

This aspect of the invention is particularly- but not exclusively- advantageous in that the present invention may be accomplished by a computer program product enabling a computer system to carry out the operations of the data processing system of the second aspect of the invention when downloaded or uploaded into the computer system. Such a computer program product may be provided on any kind of computer readable medium, or through a network.

The individual aspects of the present invention may each be combined with any of the other aspects. These and other aspects of the invention will be apparent from the following description with reference to the described embodiments.

### BRIEF DESCRIPTION OF THE FIGURES

The invention will now be described in more detail with regard to the accompanying figures. The figures show one way of implementing the present invention and is not to be construed as being limiting to other possible embodiments falling within the scope of the attached claim set.
*Figure 1**:*

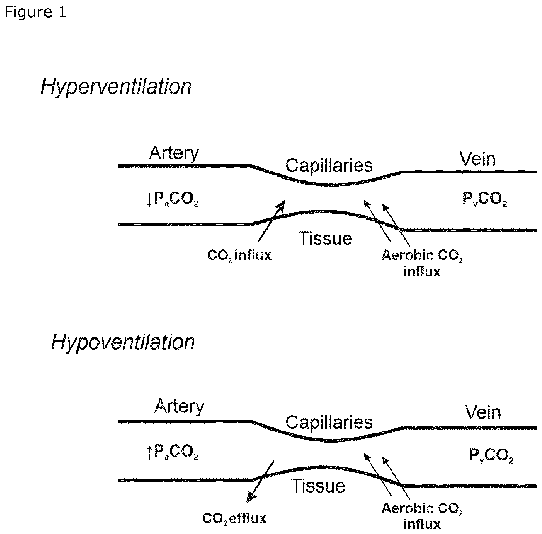

   Figure 1 shows how hyper- and hypo-ventilation impacts arterial blood and generates an overall movement of CO₂ between tissues and blood.
*Figure 2*

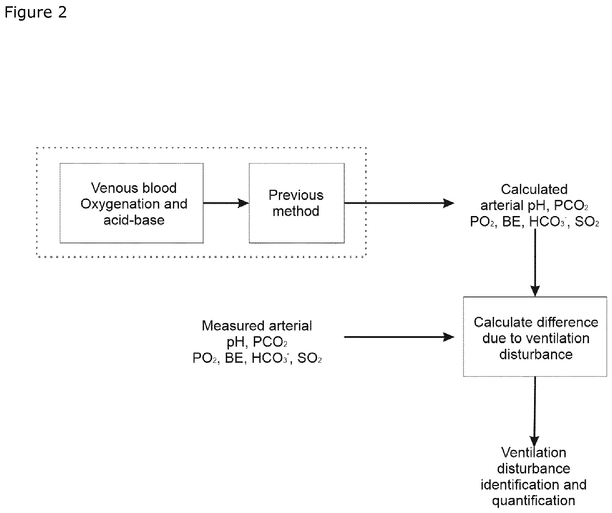

   Figure 2 is a flow chart of the method according to the present invention.
*Figure 3*

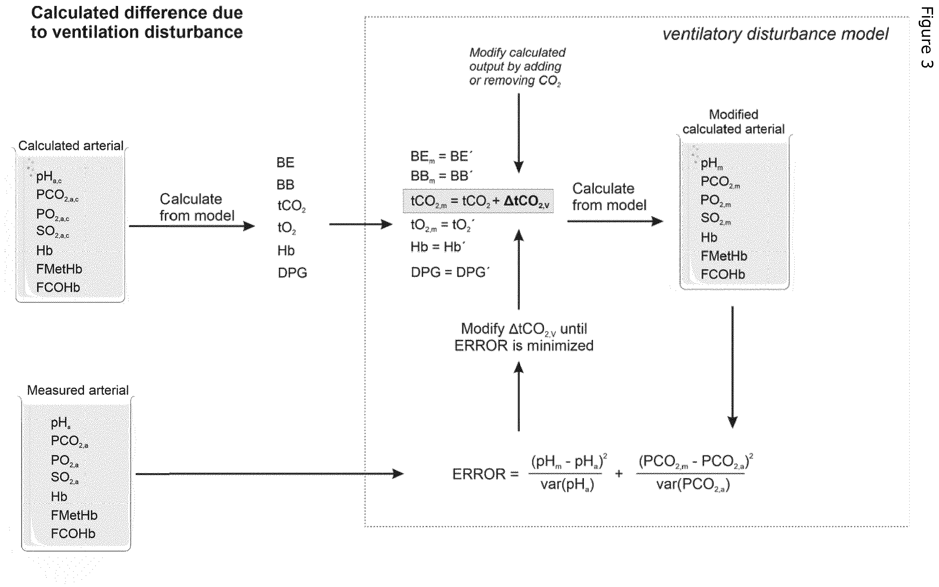

   Figure 3 illustrates an embodiment of the method using mathematical model(s) of acid-base chemistry of the blood to determine the addition/removal of CO₂ necessary to account for the effect of transient changes in ventilation.
*Figure 4*/*4a*

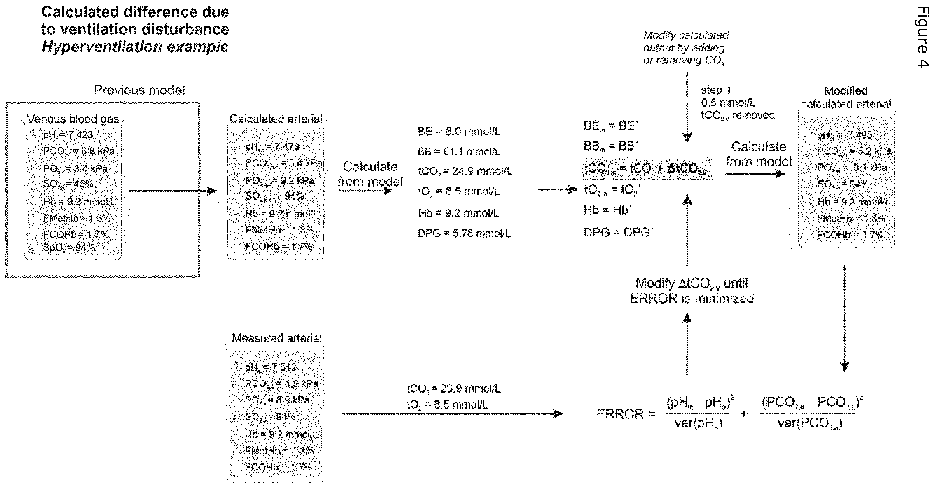

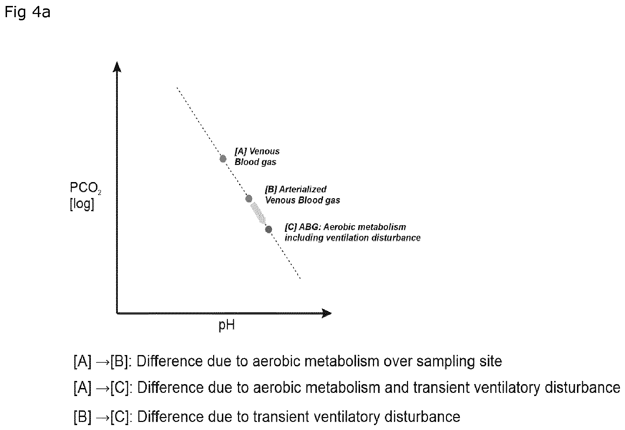

   Figure 4 illustrates a simulated patient case illustrating the calculation of the degree to which transient increases in ventilation can be described by mathematical removal of CO₂ from calculated arterial values to account for transient hyperventilation.
*Figure 5*/*5a*

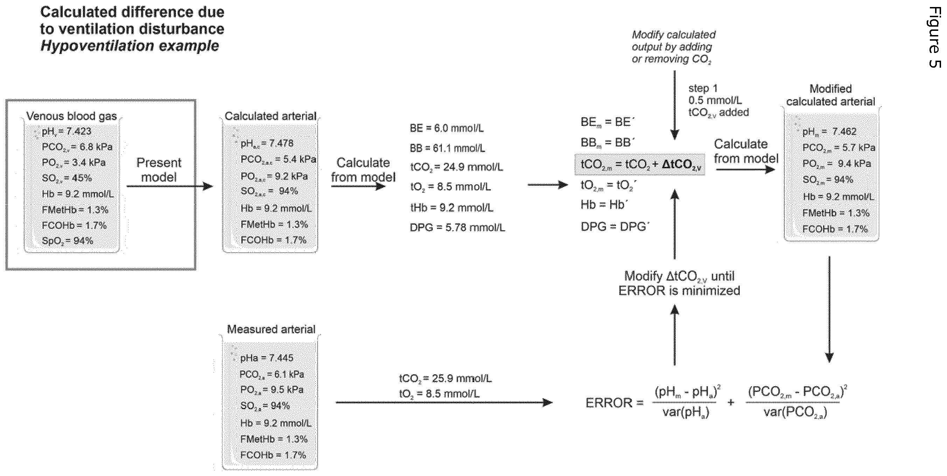

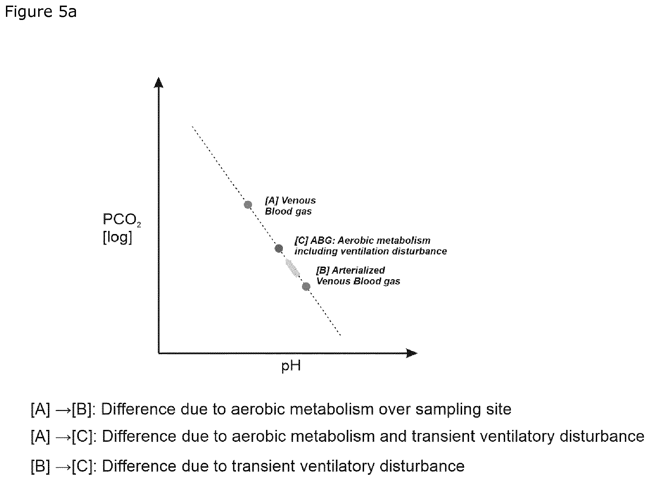

   Figure 5 illustrates a simulated patient case illustrating the calculation of the degree to which transient decreases in ventilation can be described by mathematical addition of CO₂ from calculated arterial values to account for transient hypoventilation.
*Figure 6*

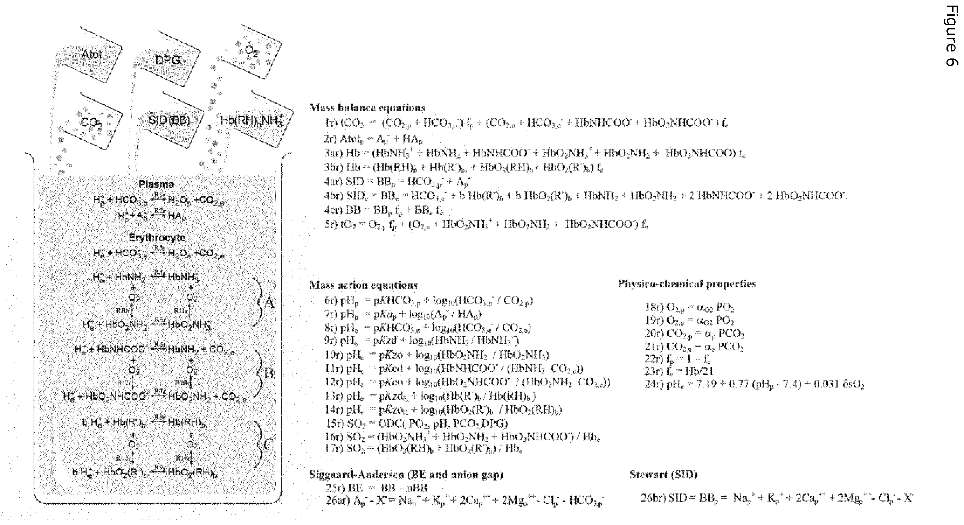

   Figure 6 illustrates an example of a mathematical model of acid-base chemistry, modified from a previous publication (7).

### DETAILED DESCRIPTION OF AN EMBODIMENT

This invention is a method and a corresponding computer system for identifying the degree to which transient changes in ventilation have modified measures of arterial acid-base status. An element of this invention is a comparison of calculated and measured values of arterial acid-base status.

Generally, it is known that there are artefacts about arterial blood influence from ventilation etc., though, the degree and quantification is poorly understood.

Particularly, the assumption of a fixed ratio of O₂ consumption and CO₂ production may be violated in the condition where the patient has a sudden, transient increase or decrease in ventilation, cf. Figure 1. A sudden increase in ventilation will result in a rapid expiration of CO₂. As arterial blood volume is relatively small, the rapid expiration of CO₂ will be seen quickly in arterial blood as a lowering of arterial PCO₂, but without substantial changes in arterial oxygen levels. As the arterial blood with lowered PCO₂ passes through the tissues, the transport of CO₂ from tissues to blood will not then solely be due to O₂ consumption and CO₂ production, and an excess transport will occur above that of aerobic metabolism, as illustrated by CO₂ influx in figure 1. Tissues, and in particular interstitial fluid, contain large quantities of CO₂. As such, a PCO₂ gradient will exist between tissue and blood, due to the transiently reduced arterial PCO₂, and the transport of CO₂ from the tissues to the blood will be elevated in relation to the use of oxygen in aerobic metabolism, as illustrated by CO₂ influx in figure 1.

A similar but opposite effect can occur in for a sudden decrease in ventilation, where the increase in arterial CO₂ can generate a PCO₂ gradient from blood to tissues and the transport of CO₂ from tissues to blood will be reduced in relation to the use of O₂ due to aerobic metabolism, and a deficit of CO₂ transport will occur as illustrated by CO₂ efflux in figure 1.

For both these cases, increase and decrease in ventilation, the application of the previously published method will not result in the calculated values of PCO₂ and pH matching measured values in the arterial blood, due to the excess or deficit of CO₂ transport between tissues to blood. This means that a mathematical simulation, adding or removing CO₂ from calculated arterial values until measured values are reached, will indicate the degree to which the arterial blood has been modified by transient changes in ventilation.

If the increase or decrease in ventilation persists, then the body will reach a steady state for CO₂. As such, the changes in PCO₂ gradient between the tissues and blood due to the transient changes in ventilation will disappear. The method described here therefore refers only to transient changes in breathing over a matter of seconds or minutes. These changes can be due to the depth or volume of breathing, or the frequency. They can occur, for example, if a patient increases breathing due to stress or if a patient holds their breath due to, for example, the fear of pain associated with an arterial puncture. The effects of these transient changes are expected to be on CO₂ alone, with no changes to the buffering or other characteristics of blood. As a consequence, they can be accounted for by addition or removal of CO₂ alone as it will be understood from the teaching and principle of the present invention.

Figure 2 is a schematic drawing of the method of the invention. The method, illustrated on the figure, includes input from a previous method calculating arterial acid-base and oxygenation status from venous blood (1,2) as defined in steps a, b, and c of the first aspect of the invention. Using that as input, along with measured arterial values, enables calculation of the effects of transient changes in ventilation on arterial acid-base status from differences between calculated and measured arterial blood values. The differences in calculated and measured arterial values describing acid-base status can be understood by several different embodiments of the method. Generally, the calculated arterial values are defined as an embodiment of the first estimated arterial blood values, and the measured arterial values are defined as an embodiment of the second reference acid-base status and oxygenation values of arterial blood in the first, second and third aspect of the present invention.

Figure 3 illustrates a preferred embodiment. Here, the calculated arterial from the previous method (1,2) is modified by a concentration of CO₂ (ΔtCO_{2,V}) needed to be added to or removed from calculated arterial acid base status so that pH and PCO₂ closely matches the measured arterial values. The value of ΔtCO_{2,V} allowing this match is then attributed to the change in concentration of CO₂ across the tissues not due to aerobic metabolism, but rather due to a transient change in ventilation, as indicated by the 'v' term in the subscript. As illustrated in the figure, the value of ΔtCO_{2,V} can be calculated by iteratively searching though possible values, until a value is found where the difference between calculated and measured arterial values of pH and PCO₂ are minimized. One way of calculating this error is illustrated on figure 3.

Figure 4 illustrates a simulated example of the method in the situation where transient hyperventilation has occurred. Values of pH and PCO₂ in the calculated arterial blood differ from those in the measured arterial sample, and removal of CO₂ is required such that modified calculated arterial values of pH and PCO₂ match measured arterial values. Illustrated in the figure is a first step in searching for the correct value of ΔtCO_{2,V}, where a value of -0.5 mmol/l has been used, and modified arterial values become closer to the measured arterial values. The value of -0.5 mmol/I ΔtCO_{2,V} would mean that at least 0.5 mmol/l of CO₂ has been added to the blood during its transit from the arteries to veins which cannot be accounted for by aerobic metabolism. This change is due to a greater tissue to blood PCO₂ gradient caused by transient hyperventilation of the arterial blood which lowers arterial PCO₂. A common way of describing CO₂ modification is through buffer lines illustrating the relationship between pH and PCO₂ in the blood. One of these is illustrated in figure 4a, illustrating this case. Venous blood (shown as a dot [A]) has higher PCO₂ and lower pH than arterial. If all transport of CO₂ over the tissues were due to aerobic metabolism, then calculated arterial values, as shown by the dot [B], would match measured values, as shown by the [C] dot. Movement from the [B] to the dot [C] is described by the need for further removal of CO₂, i.e. a negative ΔtCO_{2,V}, as described here.

Figure 5 illustrates a simulated example of the method in the situation where transient hypoventilation has occurred. Values of pH and PCO₂ in the calculated arterial blood differ from those in the measured arterial sample, and addition of CO₂ is required such that modified calculated arterial values of pH and PCO₂ match measured arterial values. Illustrated in the figure is a first step in searching for the correct value of ΔtCO_{2,V}, where a value of 0.5 mmol/l has been used, and modified arterial values become closer to the measured arterial values. The value of 0.5 mmol/I ΔtCO_{2,V} would mean that at least 0.5 mmol/l of CO₂ has been removed from the blood on its transit from the arteries to veins which cannot be accounted for by aerobic metabolism. This change is due to a reduced tissue to blood PCO₂ gradient caused by transient hypoventilation of the arterial blood, which increases arterial PCO₂. A common way of describing CO₂ modification is through buffer lines illustrating the relationship between pH and PCO₂ in the blood. One of these is illustrated in figure 5a, illustrating this case. Venous blood (shown as a dot [A]) has higher PCO₂ and lower pH than arterial. If all transport of CO₂ over the tissues were due to aerobic metabolism, then calculated values, as shown by the dot [B], would match measured values, as shown by the dot [C]. Movement from the [B] to the [C] dot is described by the need for further addition of CO₂, i.e. a positive ΔtCO_{2,V}, as described here.

Figure 6 illustrates a mathematical model of the acid-base chemistry of blood, published previously (7). This model, or a similar, is required to perform simulations of modifications of the acid-base chemistry in the blood as illustrated where it states "Calculate from model" in figures 2-5. These models are readily available with this particular model included here as an example only, hence, the skilled person will understand that other models may be implemented in the context of the present invention once the principle and teaching of the present invention is understood.

### Glossary

- 1_ABGc: First estimated or calculated arterial value
- 2_ABG: Second reference arterial value
- BB: Buffer Base
- BE: Base Excess
- DPG: 2,3-disphosphoglycerate
- FCOHb: Fraction of Carboxyhaemoglobin
- FMetHb: Fraction of Methaemoglobin
- GUI: Graphical User Interface
- Hb: Haemoglobin
- HCO₃⁻: Bicarbonate ion
- PCO₂: Partial pressure of carbon dioxide in the blood
- PO₂: Partial pressure of oxygen in the blood
- RQ: Respiration Quotient
- SO_{2AE}: Oxygen saturation in estimated arterial blood
- SO2AM: Oxygen saturation in measured arterial blood
- SpO₂: Oxygen saturation measured by pulse oximetry
- ΔtCO_{2,V}: total carbon dioxide content change due to ventilation
- tCO2: Total carbon dioxide content
- tO2: Total oxygen content
- VBG_{E}: Venous blood gas estimated
- VBG_{M}: Venous blood gas measured

### References

1. Rees SE, Toftegaard M, Andreassen S. A method for calculation of arterial acid-base and blood gas status from measurements in the peripheral venous blood. Computer Methods and Programs in Biomedicine. 2006 Jan;81(1): 18-25.
2. WO 2004/010861 (to OBI Medical Aps, Denmark)
3. Toftegaard, Rees SE, Andreassen S. Evaluation of a method for converting venous values of acid-base and oxygenation status to arterial values. Emergency Medicine Journal. 2009 Apr;26(4):268-72.
4. Rees SE, Hansen A, Toftegaard M, Pedersen J, Kristensen SR, Harving H. Converting venous acid-base and oxygen status to arterial in patients with lung disease. European Respiratory Journal, 2009, May;33(5): 1141-7.
5. Tygesen G, Matzen H, Grønkjær J, Uhrenfeldt L, Andreassen S, Gaardboe O, Rees SE. Mathematical arterialisation of venous blood in emergency medicine patients. European Journal of Emergency Medicine, 2012 Dec;19(6):363-72
6. Rees SE, Rychwicka-Kielek BA, Andersen BF, Bibi R , Pedersen JF, Weinreich UM, Birket-Smith LB, Kristensen SR. Calculating acid-base and oxygenation status during COPD exacerbation using mathematically arterialised venous blood. Clin Chem Lab Med. 2012 Dec;50(12):2149-54
7. Rees SE, Klæstrup E, Handy J, Andreassen S, Kristensen SR. Mathematical modelling of the acid-base chemistry and oxygenation of blood - A mass balance, mass action approach including plasma and red blood cells. European Journal of Applied Physiology, 2010 Feb;108(3):483-94

All of the above patent and non-patent literature are hereby incorporated by reference in their entirety.

The invention can be implemented by means of hardware, software, firmware or any combination of these. The invention or some of the features thereof can also be implemented as software running on one or more data processors and/or digital signal processors i.e. data processing on one or more computers.

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

Although the present invention has been described in connection with the specified embodiments, it should not be construed as being in any way limited to the presented examples. The scope of the present invention is to be interpreted in the light of the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention.

Furthermore, individual features mentioned in different claims, may possibly be advantageously combined, and the mentioning of these features in different claims does not exclude that a combination of features is not possible and advantageous.

## Claims

1. A computer-implemented method for determining the degree to which the arterial blood sample has been modified by the presence of a transient increase, or decrease, in ventilation of a subject, the method comprising
a) measuring and/or estimating values of blood acid-base status in a blood sample (VBG), the blood sample being obtained from venous blood of the subject,
b) providing values of measured and/or estimated arterial oxygenation (SO2AM, SO_{2AE}, SpO₂) from said subject,
c) converting the venous blood values by applying a venous-to-arterial conversion model for deriving blood acid-base status and oxygenation status into first estimated arterial blood values (1_ABG_{C}),
d) providing second reference acid-base status and oxygenation values of arterial blood (2_ABG) from the said subject,
e) implementing a ventilation disturbance model using a measure of the total carbon dioxide content (tCO₂) in the arterial blood, the said model having as input, at least, said first estimated arterial blood values (1_ABG_{C}), and said second reference values of arterial blood (2_ABG),
f) implementing the ventilation disturbance model calculating a measure indicative of the difference in the total carbon dioxide content (ΔtCO_{2,V}) between the said first estimated arterial blood values (1_ABG_{C}) and said second reference values of arterial blood (2_ABG), and
g) the ventilation disturbance model being arranged to output a measure indicative of a degree to which the arterial blood sample has been modified by the presence of a transient change in the ventilation using said measure.

2. The method according to claim 1, wherein the ventilation disturbance model is further performing a minimization process of the measure (ΔtCO_{2,V}).

3. The method according to claim 2, wherein the minimization process of the measure (ΔtCO_{2,V}) is performed by an iteration process using the measure.

4. The method according to claim 3, wherein the iteration of the ventilation disturbance model is mathematically modifying the total carbon dioxide content (tCO₂) so as to approximate the first estimated arterial blood values (1_ABG_{C}) to the second reference acid-base status and oxygenation values of arterial blood (2_ABG).

5. The method according to claim 3, wherein the iteration of the ventilation disturbance model is mathematically modifying the total carbon dioxide content (tCO₂) so as to approximate the second reference acid-base status and oxygenation values of arterial blood (2_ABG) to the first estimated arterial blood values (1_ABGc).

6. The method according to claim 2, wherein two parameters indicative of the difference between pH and PCO₂, respectively, derived from said first estimated arterial blood values (1_ABG_{C}), and said second reference values of arterial blood (2_ABG), are used for the minimization process.

7. The method according to claim 1 or 2, wherein the output measure indicative of a degree in which the arterial blood sample has been modified by the presence of a transient in the ventilation is a quantitative measure, said quantitative measure subsequently being compared with a pre-defined limit for whether a transient in the ventilation has been detected, or not.

8. A data processing system for determining a degree in which the arterial blood sample has been modified by the presence of a transient increase, or decrease, in ventilation, said data processing system comprising:
a) means for measuring and/or estimating values of blood acid-base status in a blood sample (VBG), the blood sample being obtained from venous blood of the subject,
b) means for receiving, or providing, values of measured and/or estimated arterial oxygenation (SO2AM, SO_{2AE}, SpO₂) from said subject,
c) means for converting the venous blood values by applying a venous-to-arterial conversion model for deriving blood acid-base status and oxygenation status into first estimated arterial blood values (1_ABG_{C}),
d) means for receiving, or providing, second reference acid-base status and oxygenation values of arterial blood (2_ABG) from the said subject,
e) means for implementing a ventilation disturbance model using a measure of the total carbon dioxide content (tCO₂) in the arterial blood, the said model having as input, at least, said first estimated arterial blood values (1_ABG_{C}), and said second reference values of arterial blood (2_ABG),
f) wherein the ventilation disturbance model is calculating a measure indicative of the difference in the total carbon dioxide content (ΔtCO_{2,V}) between the said first estimated arterial blood values (1_ABG_{C}) and said second reference values of arterial blood (2_ABG), and
g) the ventilation disturbance model is arranged to output a measure indicative of a degree to which the arterial blood sample has been modified by the presence of a transient change in the ventilation using said measure.

9. A computer program product enabling a computer system to carry out the operations of the data processing system of claim 8 when downloaded or uploaded into a computer system.
